# DEMANDE DE BREVET EUROPEEN

(11) **EP 0 842 669 A1**
(43) Date de publication de la demande: **20.05.1998**
(21) Numéro de dépôt: 97402691.6
(22) Date de dépôt: 10.11.1997
(51) Int. Cl.: A61L 9/01

(54) **Procédé et produits pour la désodorisation d'effluents gazeux de cuisine**

(30) Priorité: 19.11.1996 FR 9614079
(71) Demandeur: Institut Français du Pétrole, 92852 Rueil-Malmaison Cedex (FR)
(72) Inventeur: Morlec, Jean, 44600 Saint Nazaire (FR); Deschamps, André, 78590 Noisy Le Roi (FR)

(57) **Abrégé**

L'invention concerne un procédé de désodorisation d'effluents gazeux malodorants provenant d'installations de cuisine notamment de restaurants et de l'industrie agro-alimentaire, par lavage avec une solution aqueuse renfermant un sel alcalin ou alcalino terreux d'un diacide carboxylique ou d'un acide polycarboxylique, associé éventuellement à un agent tensio actif et à un diholoside.

## Description

Il est bien connu que les installations de cuisine sont souvent la source d'une gêne olfactive pour l'environnement. C'est notamment le cas des cuisines de restaurants et des installations de cuisson industrielles dans l'industrie agro-alimentaire qui rejettent des débits importants d'air présentant une odeur désagréable et tenace.

Les produits responsables de ces mauvaises odeurs sont à la fois nombreux et mal identifiés : acides organiques, esters, aldehydes plus ou moins insaturés, graisses, ...Ils peuvent générer une odeur désagréable, même à l'état de traces.

De nombreuses techniques de traitement de ces rejets ont déjà été proposées pour éliminer ces odeurs. Parmi celles-ci on citera notamment les procédés d'adsorption par exemple sur charbons actifs, les procédés d'oxydation catalytique, les procédés mettant en oeuvre des oxydants à l'état solide, tels que le permanganate de potassium, ou en solution aqueuse comme l'eau oxygénée, l'eau de Javel... Ces procédés sont généralement peu efficaces ou ne sont efficaces que pendant une durée très limitée ce qui nécessite un remplacement fréquent des agents d'épuration et rend le traitement très coûteux.

D'autres procédés font appel à l'injection dans le gaz de produits qui ont un effet masquant mais n'éliminent pas à proprement parlé les produits responsables des mauvaises odeurs. Ces procédés sont à la fois peu efficaces et coûteux en raison des quantités importantes de produits qu'il faut injecter en continu dans les gaz rejetés.

La présente invention concerne un nouveau procédé de désodorisation de ces effluents gazeux, par une technique de lavage qui est à la fois efficace et bon marché.

La présente invention décrit un nouveau procédé de désodorisation des effluents gazeux des installations de cuisson, notamment de restaurants ou de l'industrie agro-alimentaire, caractérisé en ce que l'effluent gazeux est lavé, généralement par mise en contact avec au moins une solution généralement aqueuse renfermant au moins un sel alcalin et/ou alcalino terreux d'au moins un diacide carboxylique ou d'un acide poly carboxylique présentant de préférence de 2 à 6 atomes de carbone par molécule, associé éventuellement à au moins un agent tensioactif et à au moins un diholoside de préférence en liaison 1.6.

Les acides carboxyliques utilisables selon l'invention sont par exemple des acides dicarboxyliques comme l'acide éthanedioïque (acide oxalique), l'acide hexanedioïque (acide adipique), des acides hydroxydicarboxyliques comme l'acide 2, 3 - dihydroxybutanedioïque (acide tartrique), des acides hydroxy tricarboxyliques comme l'acide 2-hydroxy-1, 2 ,3 propane tricarboxylique (acide citrique).

Ils referment de préférence de 2 à 6 atomes de carbone. L'acide oxalique est l'un des acides préférés.

Ces acides sont généralement mis en oeuvre sous forme de sels alcalins et/ou alcalino terreux, de préférence sous forme de sels de sodium ou de potassium, de manière à obtenir une solution aqueuse de lavage présentant un pH compris entre 3 et 7 et de préférence entre 4 et 6.

La quantité de sel d'acide polycarboxylique mise en oeuvre est fonction de la nature et de la concentration des composés malodorants à éliminer. La concentration en sel dans l'eau de lavage (solution) peut varier largement, par exemple entre 0,01 et 10 kg/m³, plus généralement entre 0,05 et 1 kg/m³.

De manière avantageuse, le produit actif constitué par le sel du diacide carboxylique ou d'acide polycarboxylique décrit ci-dessus, peut-être associé à un diholoside de préférence en liaison 1.6 comme le turanose ou le maltose qui renforce le caractère réducteur.

De manière avantageuse également, le produit actif (sel de diacide ou acide poly carboxylique) peut être associé à un agent tensio actif approprié, dans lequel la balance hydrophile-lipophile (HLB) est ajustée pour favoriser le passage en phase aqueuse des composés volatils et éviter le dépôt de graisses dans l'appareil de lavage. Il s'agit le plus généralement de polyalkylène oxyde et/ou polyméthylsiloxanes ou produits équivalents. Le rapport produit actif (ou sel) / agent tensio actif peut varier largement par exemple de 1 à 10 en poids, plus particulièrement de 2 à 4.

Les différents additifs entrant dans la solution de lavage peuvent être ajoutés séparément ou en mélange, à l'état solide (poudre ou pastilles) ou liquide (solution ou suspension stabilisée par un homogénéisant traditionnel à base de résine éthylacrylate par exemple).

Ils peuvent être ajoutés en continu ou en discontinu.

Le procédé de l'invention peut être mis en oeuvre dans tous les dispositifs de lavage de gaz connus de l'art antérieur comme les colonnes à garnissage, les colonnes à plateaux, les colonnes à film mince, les colonnes à bulles, les contacteurs à brouillard ou tout autre procédé assurant le meilleur contact interfacique entre le gaz à traiter et l'eau additivée.
L'appoint de l'additif peut être réalisé de manière continue, avec purge en continu d'une partie de la solution de lavage, ou bien de manière discontinue avec remplacement périodique de la solution de lavage.

### Exemple 1

L'effluent à traiter est constitué par le rejet de ventilation d'une chaîne de fabrication de plats cuisinés à partir de poisson. Le débit est de 1100 à 1300 m³/h.

Le flux d'huile mesuré à la sortie de la chaîne de préparation est de l'ordre de 3g à l'heure.

Le lavage des gaz est effectué à l'aide d'une série de buses de pulvérisation placées, selon les règles de l'art, sur le trajet de l'air à la sortie du ventilateur d'extraction. L'eau de lavage est soutirée dans une botte de décantation sous contrôle de niveau, et le reste est recyclé par une pompe. Un appoint d'eau de procédé est introduit dans l'installation en fonction du débit d'air à traiter dans un rapport compris entre 1 et 100 ml d'eau par m³ de gaz, et plus généralement avec un rapport de 2 %.

L'additif DEOWASH type OX 75 est constitué principalement d'une proportion d'oxalate acide de potassium de l'ordre de 75 % (en poids), et de QSP100 d'un diholoside cristallisable (positionné 1.6) comme le maltose par exemple.

Des tests d'olfactométrie sont réalisés sur le produit entrant dans le système de lavage et sur le produit après traitement.

La grandeur mesurée est la dilution au seuil de perception olfactive "K50". Le jury d'olfaction est sélectionné selon la norme NFX 43-101. L'olfactomètre dynamique utilisé est de type Proviron-Guigues utilisant un seul masque de trois canaux. Les dilutions testées vont de 15 à 2 500 000 en progression géométrique 2^{-0,5} différée.

Une dose d'additif DEOWASH est introduite dans le système de lavage à raison de 100 g par m³ d'eau de lavage.

Dans ces conditions le débit d'odeur à l'entrée était égal à 175 Nm3/s. Après la tour de lavage DEOWASH, le débit d'odeur a été ramené à 3 Nm³/s, soit un abattement de 98 %.

### Exemple 2

On reproduit l'essai de l'exemple 1 mais en utilisant comme additif le DEOWASH type OX 100 constitué d'oxalate acide de potassium pur, à raison de 75 g par m³ d'eau de lavage.

Dans ces conditions, le débit d'odeur après la tour de lavage a été ramené à 20 Nm³/s, soit un abattement de 88,6 %.

### Exemple 3

L'effluent à traiter est constitué par le rejet de ventilation d'un restaurant dont le débit nominal est de 5400 Nm³/h. Les effluents provenant des friteuses en particulier sont filtrés par une batterie électrostatique.

Un lavage des gaz est effectué au moyen d'une colonne à garnissage structuré avec de l'eau additionnée de l'additif DEOWASH OX 35.

L'eau de lavage est recyclée avec un débit de 2,5 m3/h en tête de colonne au travers d'une rampe d'arrosage. La hauteur de packing (garnissage) et la densité de remplissage en fonction des débits de liquide et de gaz ont été établis selon les règles de l'art.

L'additif DEOWASH type OX 35 est constitué principalement d'une proportion de sel de sodium d'acide éthanedïoque de l'ordre de 35 %, d'environ 1,5 % de polymethylsiloxane, de 2,5 % de polyalkylène oxyde, de 5 % de phosphate disodique, et de QSP100 d'un diholoside cristallisable en liaison 1-6 comme le turanose par exemple.

Une dose d'additif est introduite dans le pied de tour à raison de 500 g par m³ d'eau de lavage. L'eau de lavage est recyclée à l'aide d'une pompe centrifuge. Le mélange est renouvelé toutes les 48 heures.

Dans ces conditions le débit d'odeur à l'entrée était égal à 1045 Nm³/s. Après la tour de lavage DEOWASH, le débit d'odeur a été ramené à 90 Nm³/s, soit un abattement de 94 %.

## Revendications

1. Procédé de désodorisation d'un effluent gazeux d'installations de cuissons de restaurants ou de l'industrie agro-alimentaire caractérisé en ce que l'effluent gazeux est mis en contact avec au moins une solution renfermant au moins un sel alcalin ou alcalino-terreux d'au moins un diacide carboxylique ou d'un polyacide carboxylique.

2. Procédé selon la revendication 1 dans lequel le dit effluent est lavé par mise en contact avec au moins une solution aqueuse renfermant au moins un sel alcalin ou alcalino terreux d'au moins un diacide carboxylique ou d'un polyacide carboxylique renfermant 2 à 6 atomes de carbone par molécule.

3. Procédé selon l'une des revendications 1 et 2 dans lequel le diacide carboxylique ou l'acide polycarboxylique est associé à au moins un agent tensio actif.

4. Procédé selon l'une des revendications 1 à 3 dans lequel le diacide carboxylique ou l'acide polycarboxylique est associé à au moins un diholoside.

5. Procédé selon la revendication 4 dans lequel le diacide carboxylique ou l'acide polycarboxylique est associé à un diholoside en liaison 1.6.

6. Procédé selon l'une des revendications 1 à 5 dans lequel le diacide carboxylique ou l'acide polycarboxylique est choisi dans le groupe constitué par l'acide oxalique, l'acide adipique, l'acide tartrique et l'acide citrique.

7. Procédé selon l'une des revendications 1 à 6 dans lequel le diacide carboxylique ou l'acide polycarboxylique est mis en oeuvre sous forme d'au moins un sel alcalin ou alcalino terreux sous forme d'un sel de sodium ou de potassium, de manière à obtenir une solution aqueuse de lavage dont le pH est compris entre 3 et 7.

8. Procédé selon la revendication 7 dans lequel le pH est compris entre 4 et 6.

9. Procédé selon l'une des revendications 4 à 8 dans lequel le diholoside est le turanose ou le maltose.

10. Procédé selon l'une des revendications 1 à 9 dans lequel la concentration en sel dans la dite solution est comprise entre 0,01 et 10 kg / m³, de préférence entre 0,05 et 1 kg / m³.

11. Procédé selon l'une des revendications 1 à 10 dans lequel le dit sel est associé à un agent tensio actif.

12. Procédé selon la revendication 11 dans lequel le rapport sel / agent tensio actif est compris entre 1 et 10, de préférence 2 à 4.

13. Procédé selon l'une des revendications 11 et 12 dans lequel l'agent tensio actif est choisi dans le groupe constitué par les polyalkylène oxydes et les polyméthylsiloxanes.
